# EUROPÄISCHE PATENTSCHRIFT

(11) **EP 0 978 283 B1**
(45) Veröffentlichungstag und Bekanntmachung des Hinweises auf die Patenterteilung: **16.10.2002**
(21) Anmeldenummer: 98112821.8
(22) Anmeldetag: 10.07.1998
(51) Int. Cl.: A61K 31/505

(54) **Herstellung eines die Transplantatabstossung unterdrückenden Mittels**
Manufacture of an agent inhibiting transplant rejection
Préparation d'un agent destiné a prévenir le rejet des greffes

(43) Veröffentlichungstag der Anmeldung: 09.02.2000
(73) Patentinhaber: Werner, Ernst, 6020 Innsbruck (AT)
(72) Erfinder: Werner, Ernst, 6020 Innsbruck (AT)
(74) Vertreter: Viering, Jentschura & Partner

(56) Entgegenhaltungen:
- DE-A- 4 418 097
- S. PFEIFFER ET AL.: "Allosteric and functional effects of the potent pterin antagonist of neuronal nitric oxide synthase 4-amino-tetrahydrobiopterin" PORTLAND PRESS PROC., Bd. 15, Nr. Biology of Nitric Oxide, Part 6, 1998, Seite 15 XP002088812
- E.R.WERNER ET AL.: "Identification of the 4-amino analogue of tetrahydrobiopterin as a dihydropteridine reductase inhibitor and a potent pteridine antagonist of rat neuronal nitric oxide synthase" BIOCHEM.J., Bd. 320, Nr. 1, 1996, Seiten 193-196, XP002088813
- B.MAYER ET AL.: "Tetrahydrobiopterin Binding to Macrophage Inducible Nitric Oxide Synthase: Heme Spin Shift and Dimer Stabilization by the Potent Pterin Antagonist 4-Amino-Tetrahydrobiopterin" BIOCHEMISTRY, Bd. 36, Nr. 27, 1997, Seiten 8422-8427, XP002088814
- S. PFEIFFER ET AL.: "Allosteric modulation of rat brain nitric oxide synthase by the pterin-site enzyme inhibitor 4-aminotetrahydrobiopterin" BIOCHEM.J., Bd. 328, Nr. 2, 1997, Seiten 349-352, XP002088815
- CHEMICAL ABSTRACTS, vol. 128, no. 26, 29. Juni 1998 Columbus, Ohio, US; abstract no. 318679e, E.R.WERNER ET AL.: "Binding of pteridines to nitric oxide synthase. Clues for the mechanism of stimulation of nitric oxide synthase by tetrahydrobiopterin" Seite 239; XP002088816 & CHEM.BIOL.PTERIDINES FOLATES 1997, PROC. INT. SYMP. PTERIDINES FOLATES,1997, Seiten 669-673,
- WORALL N.K. ET AL. TRANSPLANTATION Bd. 63, Nr. 8, 27 April 1997, Seiten 1095 - 1101
- BRANDACHER, G. ET AL.: 'The 4-Amino Analogue of Tetrahydrobiopterin Efficiently Prolongs Murine Cardiac-Allograft Survival' THE JOURNAL OF HEART AND LUNG TRANSPLANTATION Bd. 20, Nr. 3, März 2001, Seiten 1 - 3

## Beschreibung

Die Erfindung betrifft Pteridine mit der folgenden Formel: 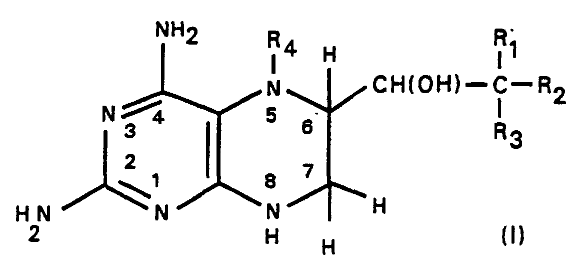 R₁, R₃ in Formel (I) stehen unabhängig voneinander für H oder OH, R₄ steht für H, CH₃, CH₂OH, CHO, R₂ steht für H, CH3, CH₂OH, CHO oder einen niederen Alkylrest (C1 bis C9), der geradkettig oder verzweigt sein kann, sowie für (CH(OH))ₙ-Y oder (CH(OH))ₙ-(CH₂)ₘ-W, wobei Y Wasserstoff oder ein niederer Alkylrest, W ein Wasserstoff oder eine Hydroxylgruppe ist, und n und m unabhängig voneinander 1-20 sind.

4-Amino Analoga von Pteridinen der Formel (I) sind neuartige Hemmer von NO Synthasen (Werner E.R. et al., Biochem. J. (1996) 320, 193-196).

Die Erfindung beruht auf der Erkenntnis, daß diese Stoffgruppe zur Herstellung eines die Immunreaktion, insbesondere die Transplantatabstoßung, unterdrückenden Arznei Mittels verwendet werden kann. Aus theoretischen Überlegungen und praktischen Versuchen ergibt sich, daß die verschiedenen Verbindungen, welche der Formel (I) entsprechen, untereinander sehr ähnliche physiologische Wirkungen aufweisen. Verantwortlich hiefür scheint die Seitenkette an C₆ mit der Formel CH(OH)-R, welche die Wirksamkeit gegenüber Verbindungen, welche an der gleichen Stelle eine Seitenkette mit der Formel CH₂-R tragen, ganz wesentlich erhöht (vgl. DE 44 18 097).

Eine einfache Verbindung, welche der Formel (I) genügt, ergibt sich, wenn R₁ und R₄ für Wasserstoff, R₂ für Methyl und R₃ für Hydroxyl steht. Die anschließend beschriebenen Versuche wurden mit dem 6-(L-*erythro*) Isomeren, also dem 2,4-Diamino-5,6,7,8-tetrahydro-6-(L*-erythro*-1,2dihydroxypropyl)pteridin ausgeführt, welches in der Beschreibung kurz als Tetrahydroaminobiopterin bezeichnet ist.

Zusätzlich zu in der klinischen Praxis eingesetzten lmmunsuppressiva zur Verhinderung der Transplantatabstoßung wie z.B. Cyclosporin A oder Corticosteroiden wurde in Tiermodellen bereits versucht, durch Hemmung der NO Synthase eine Vermeidung der Abstoßung des Transplantates zu erzielen. Grundlage dafür bildete die Erkenntnis, daß in der Abstoßung vermehrte Biosynthese von Stickstoffmonoxid festgestellt werden kann. Allerdings sind die Berichte über Effekte von NO Synthasehemmem in Tiermodellen der Transplantatabstoßung widersprüchlich. Berichten, nach denen NO Synthasehemmer für diesen Zweck geeignet sind (z.B. Worall NK et al., Transplantation 63:1095-1101 (1997)) stehen Berichte über schädliche Wirkungen gegenüber: So ist z.B. bekannt, daß Hemmung der NO Synthase in einem Maus-Modell der Reaktion des transplantierten Knochenmarkes gegen den Empfänger (graft versus host disease) zu erhöhtem Gewichtsverlust und verringertem Überleben führt (Drobyski W.R. et al., Blood 84:2363-2372, (1994)). Weiters wird berichtet, daß NO Synthasehemmung das Überleben von transplantierten Herzen in der Ratte verringert (Paul L.C. et al., Transplantation 62:1193-1195 (1996)).

Aus dem Gesagten ergibt sich, daß die Eignung des eingangs definierten Wirkstoffes zur Verhinderung der Transplantatabstoßung sich keineswegs bereits aus dessen Eigenschaft als Hemmer der NO Synthase ergibt. Es ist nicht einmal gesichert, ob der Wirkstoff überhaupt durch Hemmung der NO Synthase oder durch einen anderen Mechanismus seine Wirkung ausübt. Nicht vorauszusehen war vor allem das Ausmaß der Wirksamkeit, das durch die nachfolgend beschriebenen Tierversuche belegt ist.

### Beispiel der Wirkung von Tetrahydroaminobiopterin in einem Tiermodell der Transplantatabstoßung

Zur Untersuchung der Wirkung von Tetrahydroaminobiopterin wurde ein allogenes Herztransplantationsmodell mittel Cufftechnik verwendet (beschrieben von Brandacher et al. Acta Chir. Austriaca Suppl Nr. 130, 1997, Abstract. Nr. 77). Dabei werden Herzen von männlichen C57Bl/10 Mäusen auf männliche C3H/He Mäuse übertragen und das von außen sichtbare Schlagen des zusätzlich transplantierten Herzens beobachtet. Die Auswertung der immunsuppressiven Wirkung erfolgt durch Zählen der Tage, wie lange das zusätzliche Herz zu schlagen im Stande ist. Gruppen von 5 Tieren (Dosis 0: 3 Tiere) wurden nach Transplantation 7 Tage lang mit 0, 20, 100 und 200 mg/kg Tetrahydroaminobiopterin (als dihydrochlorid; 6R:6S ratio = 60:40) behandelt. Die Gabe des Tetrahydroaminobiopterin erfolgte einmal pro Tag als intramusculäre Injektion der Verbindung gelöst in 100 µl physiologischer Kochsalzlösung. Tabelle 3 zeigt die beobachteten Transplantatüberlebenszeiten:

**Tabelle 3:**

| Überleben von allogenen Herztransplantaten in der Maus in Abhängigkeit von der Dosis Tetrahydroaminobiopterin: | | |
|---|---|---|
| Dosis (mg kg⁻¹ d⁻¹) | Tiere/Gruppe | Transplantatüberleben (Tage) |
| 0 | 3 | 7, 8, 7 |
| 20 | 5 | 10, 12, 10, 8, 10 |
| 100 | 5 | 11, 13, 11, 13, 13 |
| 200 | 5 | 11, 13, 14, 12, 13 |

Auswertung mittels Spearman-Rank-Korrelation ergibt eine hochsignifikante Verlängerung des Transplantatüberlebens mit steigender Dosis Tetrahydroaminobiopterin (P < 0.0008)

Die angegebene Dosierung dient lediglich als Beispiel, andere pharmakologische Applikationen (z.B. intravenös, oral, Gaben mehrmals pro Tag, kontinuierliche Zugabe mittels einer Pumpe, dem Stand der Technik entsprechende Depotpräparationen) sind ebenfalls möglich.

## Patentansprüche

1. Verwendung eines Wirkstoffes mit der Formel (I) einschließlich seiner tautomeren und stereoisomeren Formen und deren Salzen 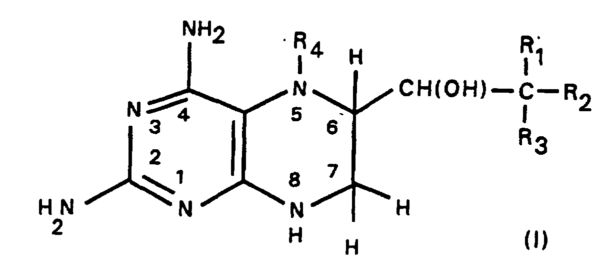 R₁, R₃ in Formel (I) stehen unabhängig voneinander für H oder OH, R₄ steht für H, CH₃, CH₂OH, CHO, R₂ steht für H, CH₃, CH₂OH, CHO oder einen niederen Alkylrest (C1 bis C9), der geradkettig oder verzweigt sein kann, sowie für (CH(OH))ₙ-Y oder (CH(OH))ₙ-(CH₂)ₘ-W, wobei Y Wasserstoff oder ein niederer Alkylrest, W ein Wasserstoff oder eine Hydroxylgruppe ist, und n und m unabhängig voneinander 1-20 sind, zur Herstellung eines Arznei mittels zur Unterdrückung der Immunreaktion, insbesondere der Transplantatabstoßung,

2. Verwendung eines Arznei- Mittels nach Anspruch 1, mit der Maßgabe, daß R₁ und R₄ für Wasserstoff, R₂ für Methyl und R₃ für Hydroxyl steht.

3. Verwendung eines Arznei- Mittels nach Anspruch 2, mit der Maßgabe, daß das Mittel das 6-(L-erythro) Isomere, also 2,4-Diamino-5,6,7,6-tetrahydro-6-(L-*erythro*-1,2 dihydroxypropyl)pteridin ist.

## Claims

1. Use of an agent having the formula (I) including its tautomeric and stereoisomeric forms and the salts thereof 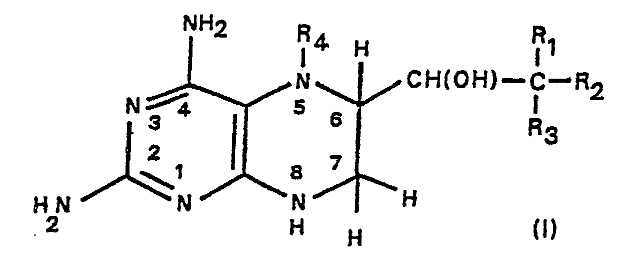 R₁, R₃ are independently from each other H or OH, R₄ is H, CH₃, CH₂OH, CHO, R₂ is H, CH₃, CH₂OH, CHO or a lower alkyl radical (C1 to C9), which can be straight-chained or branched, as well as (CH(OH))ₙ-Y or (CH(OH))ₙ-(CH₂)ₘ-W, wherein Y is hydrogen or a lower alkyl radical, W is a hydrogen or a hydroxyl group, and n and m are independently from each other 1-20 for the manufacture of a pharmaceutical for suppression of an immune reaction, in particular of graft rejection.

2. The use of a pharmaceutical of claim 1 with the proviso that R₁ and R₄ are hydrogen, R₂ is methyl and R₃ is hydroxyl.

3. The use of a pharmaceutical of claim 2 with the proviso that the agent is the 6-(L-erythro) isomer, i.e. 2,4-diamino-5,6,7,8-tetrahydro-6-(L-erythro-1,2-dihydroxypropyl)pteridine.

## Revendications

1. Utilisation d'un composé de formule I, y compris ses formes tactomères et stéréoisomères ainsi que ses sels : 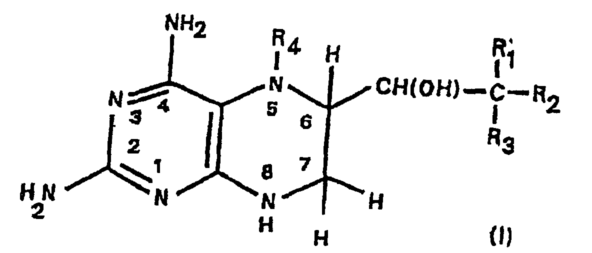 dans laquelle R₁, R₃ représentent indépendamment l'un de l'autre H ou OH et R₄ représente H, CH₃, CH₂OH, CHO, R₂ représente H, CH₃, CH₂OH, CHO ou un résidu alkyle inférieur en C1-C9 qui peut être à chaîne droite ou ramifiée, ainsi que (CH(OH))ₙ- Y ou (CH(OH))ₙ - (CH₂)ₘ -W, où Y représente un hydrogène ou un reste alkyle inférieur, W représente un hydrogène ou un groupe hydroxyle et n et m représentent indépendamment l'un de l'autre un nombre de 1 à 20, pour la préparation d'un médicament pour abaisser la réaction immune, en particulier le rejet de greffe.

2. Utilisation d'un médicament selon la revendication 1 avec cette condition que R₁ et R₄ représentent l'hydrogène, R₂ représente le méthyle et R₃ représente un hydroxyle.

3. Utilisation d'un médicament selon la revendication 2 avec cette condition que ce produit soit l'isomère 6-(L-érythro), c'est à dire la 2-4-diamino-5,6,7,8-tétrahydro-6-(L-erythro-1,2 dihydroxypropyl)pteridine.
